(19) Europäisches Patentamt
European Patent Office
Office européen des brevets

(11) **EP 1 115 364 B1**

(12) # FASCICULE DE BREVET EUROPEEN

(45) Date de publication et mention de la délivrance du brevet:
**08.12.2004 Bulletin 2004/50**

(21) Numéro de dépôt: **99934817.0**

(22) Date de dépôt: **30.07.1999**

(51) Int Cl.[7]: **A61K 6/10**, C08L 83/04, C08K 5/00

(86) Numéro de dépôt international:
**PCT/FR1999/001885**

(87) Numéro de publication internationale:
**WO 2000/007546 (17.02.2000 Gazette 2000/07)**

(54) **SYSTEME ELASTOMERE SILICONE AYANT DES PROPRIETES BIOCIDES UTILISABLE NOTAMMENT POUR LA PRISE D'EMPREINTES DENTAIRES**

SILIKONELASTOMER-SYSTEM MIT BIOZIDEN EIGENSCHAFTEN FÜR DIE VERWENDUNG ALS ABFORMMATERIAL

SILICONE ELASTOMER SYSTEM HAVING BIOCIDE PROPERTIES USEFUL IN PARTICULAR FOR MAKING IMPRESSIONS IN DENTISTRY

(84) Etats contractants désignés:
**AT BE CH CY DE DK ES FI FR GB GR IE IT LI LU MC NL PT SE**

(30) Priorité: **31.07.1998 FR 9810023**

(43) Date de publication de la demande:
**18.07.2001 Bulletin 2001/29**

(73) Titulaire: **RHODIA CHIMIE**
**92408 Courbevoie Cédex (FR)**

(72) Inventeurs:
- **PUSINERI, Christian**
  **F-69360 Serezin du Rhône (FR)**
- **DEL TORTO, Marco**
  **I-21058 Solbiate Olona (IT)**

(74) Mandataire: **Trolliet, Maurice et al**
**RHODIA SERVICES**
**Direction de la Propriété Industrielle**
**CRIT-Carrières - BP 62**
**69192 Saint-Fons Cédex (FR)**

(56) Documents cités:
**EP-A- 0 265 776**
**EP-A- 0 361 301**
**EP-A- 0 493 186**
**FR-A- 2 707 660**
**FR-A- 2 750 598**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

Le domaine de la présente invention est celui des systèmes organosiliciques comportant une composition polyorganosiloxane durcissable en un élastomère ainsi qu'au moins un principe actif biocide choisi parmi les dérivés du chlore. Les applications visées par de tels systèmes sont, notamment, la prise d'empreintes et, plus particulièrement encore, la prise d'empreintes dentaires dans le cadre de réalisation de prothèses.

La présente invention concerne donc un système élastomère silicone ayant des propriétés biocides et utilisable, notamment, pour la prise d'empreintes, par exemple, dentaires.

L'invention a également pour objet un matériau pour la prise d'empreintes notamment dentaires comprenant le susdit système élastomère silicone.

Enfin, l'invention vise l'utilisation du système ou du matériau pour la prise d'empreintes, par exemple dentaires, de même qu'un procédé de préparation dudit système et/ou dudit matériau.

La notion d'activité biocide renvoie aux domaines de l'hygiène, du confort et des applications médicales et paramédicales. L'utilisation des élastomères silicones est largement répandue dans ces domaines. Ceci est dû en partie au fait que les élastomères silicones offrent, d'une part une grande diversité de caractéristiques chimiques, mécaniques et physiques, et, d'autre part, un caractère non toxique, non irritant et non allergisant. En outre, les élastomères silicones constituent de piètres substrats de culture pour les micro-organismes, ce qui leur confère des aptitudes remarquables au regard de l'hygiène.

On sait par ailleurs que les silicones du type élastomère vulcanisable à froid se présentant avantageusement sous forme de deux composants (EVF 2 ou RTV 2) sont particulièrement adaptés à l'art consistant à prendre des empreintes, à reproduire et à créer des formes. Ceci s'explique par le fait que ces silicones sont doués de propriétés de fluidité et de filmogénéité avant réticulation, ce qui rend possible la prise d'une empreinte de n'importe quel modèle et de n'importe quelle forme. La réticulation subséquente qui entraîne le durcissement de l'élastomère silicone permet de constituer des moules adaptés à partir des empreintes. La bonne tenue thermique des EVF (ou RTV) réticulés permet aux moules réalisés à partir de ces matériaux, de résister aux hautes températures de fusion de certains matériaux de moulage tels que les métaux.

La réalisation de moules en élastomère silicone EVF ou RTV est particulièrement intéressante pour obtenir des petites séries pour lesquelles le coût et la durée de confection du moule ne sont pas prohibitifs, contrairement à ce que l'on observe lorsque le moule est métallique.

Il existe un lien entre cette application "moulage" des EVF ou RTV et leurs emplois dans le domaine de la santé, puisque les dentistes, les podologues et les chirurgiens esthéticiens font appel à ces élastomères silicones pour réaliser des moulages de dentiers ou de formes correctrices (intercalaires, prothèses mammaires).

Ainsi, sans que cela ne soit limitatif, les EVF ou RTV sont particulièrement intéressants dans le domaine des prises d'empreintes notamment dentaires, car ils sont disponibles à l'état non réticulé sous des rhéologies de type fluides a pâteuse. Par ailleurs, ils réticulent en quelques minutes à température ambiante; ils sont non toxiques et satisfont aux réglementations européennes en matière pharmaceutique. On peut citer la demande de brevet français publiée sous le nº 2 750 598, qui porte sur une composition à base de silicone pour empreintes dentaires. Toutefois, les prises d'empreintes restent sujettes à des contaminations en surface, voire dans la masse, par des micro-organismes pathogènes et deviennent ainsi des vecteurs de propagation et de dissémination de microbes entre le cabinet du dentiste et le laboratoire du prothésiste. Pour tenter de remédier à cela, on peut procéder classiquement à un traitement de désinfection de l'empreinte par mise en contact de celle-ci avec un antiseptique conventionnel, par exemple par trempage ou par pulvérisation. Ce type de traitement de désinfection limitée à la surface n'est certes pas dénué totalement d'efficacité dans la tentative recherchée de rupture de la chaîne de contamination, mais cela reste tout à fait insuffisant. En outre, cette étape supplémentaire de désinfection de surface représente une contrainte dont s'affranchirait volontiers les praticiens.

Dans le cadre d'une recherche d'efficacité en matière de décontamination de ces matériaux de prise d'empreintes dentaires notamment, on connaît également la solution consistant à incorporer dans le matériau lui-même, un antiseptique qui assurera sa fonction première, à la fois dans la masse et en surface. A titre d'illustration de cette proposition technique, on peut citer la demande de brevet européen EP n° 0 361 301 qui décrit l'introduction d'un biocide dans un matériau pour prise d'empreintes dentaires à base d'alginate.Les alginates sont une alternative peu convaincante aux EVF ou RTV. L'agent biocide concerné se présente sous la forme d'une solution aqueuse de sels d'ammonium quaternaire et de composés à base de guanidine et de ses dérivés. Cette solution remplace l'eau nécessaire à la préparation de la pâte d'alginate.

On connaît également en dehors de l'application spécifique "prise d'empreintes dentaires" mais toujours dans le cadre des élastomères silicones biocides utilisés dans le domaine de l'hygiène et de la santé, la demande de brevet européen EP-A-0 493 186 qui décrit un système organosilicique biocide comprenant :

- une composition organopolysiloxane bicomposante, réticulante à température ambiante ou à la chaleur, par des

réactions notamment de polyaddition ,

- ainsi qu'un système biocide constitué par un sel d'un hydracide ou d'un oxacide minéral ou d'un acide organique dérivé d'un biguanide polymère linéaire : poly(hexaméthylène-biguanide). Cet élastomère silicone à activité biocide est destiné à être incorporé dans des éponges pour lutter contre la prolifération bactérienne, tant au cours de la période de stockage que pendant la durée d'utilisation desdites éponges.

Il convient également de citer dans cette revue de l'art antérieur, la demande de brevet français 93 08 114 publiée sous le n° 2 707 660 et qui concerne un système élastomère silicone obtenu par réticulation d'une composition comprenant :

A - 100 parties en poids d'un silicone $\alpha,\omega$-di(hydroxy)-diorganopolysiloxane;
B - de 2 à 20 parties en poids d'un silane réticulant renfermant un groupe hydrolysable;
C - de 0 à 150 parties en poids d'une charge minérale ou organique;
D - 1 à 150 parties en poids pour 100 parties de A + B + C, d'un composé minéral ou organique solide, susceptible de libérer du chlore actif au contact de l'eau ou de l'humidité (de préférence hypochlorite de calcium);
E - éventuellement un catalyseur de réticulation par polycondensation.

Cette composition contient au moins 0,01% de son poids d'eau apportée et éventuellement générée par voie intrinsèque et éventuellement apportée par voie extrinsèque. Dans cette demande de brevet, on préconise l'utilisation d'un tel système comme agent de libération de chlore actif, notamment pour le traitement de l'eau.

Le système silicone élastomère selon cette demande de brevet est strictement limité au silicone de type $\alpha$, $\omega$-di(hydroxy)diorganopolysiloxane réticulable par polycondensation En outre, le système élastomère silicone divulgué dans cette demande de brevet est décrit comme pouvant être utilisé pour la réalisation de joints ou de films utiles dans les domaines d'hygiène et des applications sanitaires. Une autre application envisagée dans cette référence antérieure est celle du traitement de l'eau. Le système se présente alors sour forme de matrice silicone contenue dans une cartouche qui libère progressivement le principe actif biocide hypochlorite de calcium. Il n'est nullement question, dans cette demande de brevet, d'application du système à titre de matériau pour la prise d'empreintes notamment dentaires. Il s'est avéré que les antiseptiques mis en oeuvre dans la masse des matériaux tels que décrits dans l'EP-A-0 361 301, dans l'EP-A-0 493 186 et dans le FR-A-2 707 660 ne satisfont pas au cahier des charges propre aux applications des matériaux élastomères dans le domaine de l'hygiène et de la santé et, plus particulièrement mais non limitativement, dans le cas des matériaux pour prise d'empreintes notamment dentaires. Le cahier des charges considéré comprend entre autres les spécifications suivantes :

- le matériau chargé en biocide doit être compatible pour le contact avec la peau et les muqueuses, en particulier buccales : il doit être non toxique, non allergisant et non irritant aux doses d'emploi;
- le matériau doit contenir une teneur suffisante en biocide pour pouvoir développer en surface l'activité antiseptique recherchée quelque soit le mécanisme d'action : activité de contact et/ou libération de faibles quantits de produit actif;
- l'agent biocide ne doit pas développer d'activité inhibitrice vis-à-vis des catalyseurs de réticulation, en particulier vis-à-vis des catalyseurs au platine dans le cas du système EVF ou RTV de polyaddition;
- la présence d'agent biocide dans le matériau ne doit pas interférer et nuire aux performances techniques du matériau élastomère; en particulier, "la réticulabilité", le temps de prise, les propriétés rhéologiques avant réticulation, les propriétés mécaniques après réticulation, la stabilité dimensionnelle et la tenue thermique, ne doivent pas être affectées.

Dans un tel contexte technique, l'un des objectifs essentiels de la présente invention est de fournir un système élastomère silicone à propriétés biocides et utilisable notamment pour la prise d'empreintes, par exemple dentaires, ledit système se devant d'être autant que faire se peut, conforme au cahier des charges susmentionné.

Un autre objectif essentiel de l'invention est de fournir un système élastomère silicone biocide, notamment de type EVF 2 ou RTV 2, qui soit simple à obtenir, économique, performant sur le plan de l'activité antiseptique et possédant d'excellentes propriétés physiques et chimiques d'utilisation.

Un autre objectif essentiel de l'invention est de proposer un matériau pour la prise d'empreintes dentaires notamment satisfaisant aux spécifications susvisées pour le système.

Un autre objectif essentiel de l'invention est de proposer l'utilisation d'un système élastomère silicone biocide pour la prise d'empreintes, notamment dentaires.

Un autre objectif essentiel de la présente invention est de fournir un procédé de préparation du système susévoqué qui soit industriel et peu onéreux.

Pour atteindre ses objectifs, parmi d'autres, les inventeurs ont eu le mérite de mettre à jour de manière tout

à fait surprenante et inattendue et après de longues et laborieuses recherches et expérimentations, une classe judicieusement sélectionnée d'agents biocides promoteurs de chlore actif.

D'où il s'ensuit que la présente invention concerne un système élastomère silicone, utilisable notamment par la prise d'empreintes, notamment, dentaires ayant des propriétés biocides et caractérisé en ce qu'il comprend essentiellement :

(I) au moins une composition polyorganosiloxane (POS) réticulable ou réticulée sous forme d'élastomère, et comportant au moins un catalyseur de réticulation,
(II) et au moins un agent biocide.

Sont considérés comme "biocides" au sens de l'invention, les agents doués de propriétés destructrices vis-à-vis de micro-organismes vivants de n'importe quel type : bactéries, virus, champignons, levures, sous forme végétative (spores) ou non.

Outre la sélection avantageuse de l'agent biocide parmi les précurseurs de chlore actif, l'invention repose également sur le choix qualitatif et quantitatif des élastomères silicones compatibles. La combinaison synergique de la composition POS I et de l'agent biocide II conduit à un système ou à un matériau élastomère silicone pourvu des avantages suivants :

- antiseptique de manière stable dans le temps,
- doté de toutes les propriétés intéressantes des élastomères non-réticulés (fluidité, filmogénicité) et des élastomères réticulés (dureté, stabilité dimensionnelle, tenue thermique);
- faible prix de revient, en particulier pour réaliser des moules industriels.

La composition POS (I) comporte des POS réticulables par polyaddition et par ailleurs la concentration $C_{II}$ en agent biocide, exprimée en % en poids par rapport à la masse totale (I + II), est :

$$C_{II} \leq 1$$

de préférence

$$C_{II} \leq 0,8$$

et plus préférentiellement encore

$$0,001 \leq C_{II} \leq 0,5.$$

Il est particulièrement intéressant de noter qu'à ces faibles concentrations l'agent biocide sélectionné selon l'invention est parfaitement performant quant à l'activité antiseptique, sans pour autant nuire à la réactivité des EVF ou RTV de polyaddition formant l'élastomère silicone.

La composition POS (I) est une composition de silicone polyaddition durcissable en un élastomère par des réactions d'hydrosilylation, caractérisée en ce qu'elle comporte :

($I_a$) - au moins une huile diorganopolysiloxane présentant, par molécule, au moins deux groupes alcényle, de préférence vinyle, liés au silicium;
($I_b$) - au moins une huile diorganopolysiloxane présentant par molécule au moins trois atomes d'hydrogène liés au silicium,
($I_c$) - une quantité catalytiquement efficace d'un catalyseur qui est en général un composé d'un métal du groupe du platine.

Les quantités de ($I_a$) et ($I_b$) sont généralement choisies de manière que le rapport molaire des atomes d'hydrogène liés au silicium dans ($I_b$) sur les radicaux vinyle liés au silicium dans ($I_a$), soit compris généralement entre 0,4 et 10, de préférence entre 0,6 et 5. Ce rapport peut être toutefois compris entre 2 et 5, si on souhaite faire des mousses d'élastomères.

Les groupes alcényles dans ($I_a$) et les atomes d'hydrogène dans ($I_b$) sont généralement liés à des atomes de silicium différents.

Ces compositions réticulent par réaction d'addition (dénommées également réaction d'hydrosilylation), catalysée par un composé d'un métal d'un groupe du platine, d'un groupe alcényle de l'organopolysiloxane ($I_a$) sur une fonction hydrure de l'organopolysiloxane ($I_b$).

Les catalyseurs ($I_c$) sont également bien connus. On utilise de préférence les composés du platine et du rhodium. On peut utiliser les complexes du platine et d'un produit organique décrit dans les brevets américains US-A-3 195 601, US-A-3 159 602, US-A-3 220 972 et les brevets européens EP-A- 57 459, EP-A- 188 978 et EP-A- 190 530, les complexes du platine et d'organopolysiloxane vinylé décrits dans les brevets américains US-A- 3 419 593, US-A-3 715 334, USA-A-3 377 432 et US-A-3 814 730.

On peut également mettre en oeuvre les complexes du rhodium décrits dans les brevets britanniques : GB-A- 1 421 136 et GB-A-1 419 769.

Les catalyseurs au platine sont préférés. Dans ce cas la quantité pondérale de catalyseur ($I_c$) calculée en poids de platine-métal est généralement comprise entre 2 et 600 ppm, en général entre 5 et 200 ppm basés sur le poids total des organosiloxanes ($I_a$) et ($I_b$).

Les compositions de polyaddition dans le cadre de la présente invention sont celles qui comportent :

($I_a$) : au moins une huile diorganopolysiloxane bloquée à chaque extrémité de sa chaîne par un motif vinyldiorganosiloxyle dont les radicaux organiques liés aux atomes de silicium sont choisis parmi les radicaux méthyle, éthyle et phényle, au moins 60% molaire de ces radicaux étant des radicaux méthyle, de viscosité 100 à 500 000, de préférence de 1 000 à 200 000 mPa.s à 25°C;

($I_b$) : au moins un organohydrogénopolysiloxane choisi parmi les homopolymères et copolymères liquides linéaires ou en réseau présentant par molécule au moins 3 atomes d'hydrogène liés à des atomes de silicium différents et dont les radicaux organiques liés aux atomes de silicium sont choisis parmi les radicaux méthyle, éthyle et 60% au moins de ces radicaux étant des radicaux méthyle, le produit ($I_b$) étant utilisé en quantité telle que le rapport molaire des fonctions hydrure sur les groupes vinyle sit compris entre 1,1 et 4:

($I_c$) : une quantité catalytiquement efficace d'un catalyseur au platine.

De façon préférée, jusqu'à 50% en poids du polymère $I_a$ est remplacé par un copolymère en réseau comportant les motifs triméthylsiloxyle, méthylvinylsiloxyle et $SiO_{4/2}$ dans lequel de 2,5 à 10% molaire des atomes de silicium comportent un groupe vinyle et dans lequel le rapport molaire des groupes triméthylsiloxyle au groupe $SiO_{4/2}$ est compris entre 0,5 et 1.

Suivant une disposition avantageuse de l'invention la composition POS (I), comprend en outre un ou plusieurs additifs fonctionnels ($I_d$) choisis dans le groupe comprenant :

- les inhibiteurs de la réaction de réticulation par polyaddition, de préférence les inhibiteurs du catalyseur au platine;
- les charges renforçantes et/ou semi-renforçantes et/ou de bourrage,
- les agents plastifiants,
- et leurs mélanges.

Les inhibiteurs sont des composés bien connus. On peut en particulier utiliser les amines organiques, les silazanes, les oximes organiques, des diesters de diacide carboxylique, les alcools acétyléniques, les cétones acétyléniques, les vinylméthylcyclopolysiloxanes (voir par exemple US-A-3 445 420 et US-A-3 989 667). L'inhibiteur est utilisé à raison de 0,005 à 5 parties en poids, de préférence de 0,01 à 3 parties en poids à 100 parties du constituant ($I_a$).

Les charges renforçantes ou semi-renforçantes ou de bourrage sont, de préférence, choisies parmi les charges siliceuses.

Les charges siliceuses renforçantes sont choisies, de préférence, parmi les silices de combustion et les silices de précipitation. Elles ont une surface spécifique, mesurée selon les méthodes BET, d'au moins 50 $m^2/g$, de préférence supérieure à 70 $m^2/g$, une dimension moyenne des particules primaires inférieures à 0,1 μm (micromètre) et une densité apparent inférieure à 200 g/litre.

Ces silices peuvent être incorporées telles quelles ou après avoir été traitées par des composés organosiliciques habituellement utilisés pour cet usage. Parmi ces composés figurent les méthylpolysiloxanes tels que l'hexaméthyldisiloxane, l'octaméthyldisiloxane, l'octaméthylcyclotétrasiloxane, des méthylpolysilazanes tels que l'hexaméthyldisilazane, l'hexaméthylcyclotrisilazane, des chorosilanes tels que le diméthylchlorosilane, le triméthylchlorosilane, le méthylvinyldichlorosilane, le diméthylvinylchlorosilane, des alcoxysilanes tels que le diméthyldiméthoxysilane, le diméthylvinyléthoxysilane, le triméthylméthoxysilane.

Lors de ce traitement, les silices peuvent accroître leur poids de départ jusqu'à un taux de 20% de préférence 18% environ.

Les charges semi-renforçantes ou de bourrage ont un diamètre particulaire supérieur à 0,1 μm et sont choisies de préférence parmi le quartz broyé, les argiles calcinées, les terres de diatomées et les silicates d'aluminium et/ou

de sodium.
Les charges semi-renforçantes ou de bourrage peuvent également être constituées par des charges à base d'alumine et d'oxyde de titane.

Sur le plan pondéral, les charges peuvent représenter de 5 à 100 parties en poids, de préférence de 5 à 50 parties en poids pour 100 parties de la somme des POS $I_a$ ou ($I_{a'}$) + $I_b$ ou ($I_{b'}$)

Les agents plastifiants susceptibles de constituer des additifs additionnels ($I_d$) peuvent être par exemple l'huile de vaseline et/ou une paraffine.

Les compositions POS (I) plus spécialement envisagées dans le cadre de l'invention sont des compositions à deux composantes ou à deux emballages, également dénommées compositions bi-composantes A-B. Ce sont des précurseurs des élastomères réticulés à température ambiante ou à la chaleur pour accélérer la réaction. Cette réticulation est obtenue par mélange de composants A et B, isolément non durcissables.

Pour les compositions POS (I) de polyaddition, il est souhaitable qu'elles comprennent un inhibiteur de réticulation dès lors qu'elles sont sous forme de compositions bi-composantes en deux emballages. En effet, l'inhibition apporte une temporisation qui permet d'obtenir une bonne homogénéisation du mélange constituant le système EVF 2 ou RTV 2//biocide selon l'invention.
Pour obtenir une bonne homogénéisation de répartition de la matière active, il est en effet souhaitable que la matrice silicone présente une certaine viscosité de l'ordre de 5 000 à 30 000 mPa.s à 25°C.

Une telle viscosité peut être obtenue par une préréticulation, celle-ci étant bloquée à la viscosité désirée par addition d'un inhibiteur. On dispose ainsi de suffisamment de temps pour bien homogénéiser la matière active au sein de la matrice silicone.

La réticulation est alors achevée par chauffage de la matrice à une température telle que l'inhibiteur n'a plus d'effet sur l'action catalytique du platine.

En ce qui concerne l'agent biocide (II) mis en oeuvre dans le système selon l'invention, il est à noter qu'il comporte une chloramine, et qu'il est, de préférence, choisi dans le groupe de précurseur de chlore actif à base de composés n-chlorés comprenant :

- la chloramine B (sodium-N-chlorobenzène sulfonamide),
- la dichloroamine T (N,N-dichloro-p-toluène sulfonamide),
- la N-trichlorométhylmercapto-4-cyclohexène-1,2-dicarboxylamide),
- l'halazone (acide benzoïque p-n-dichlorosulfonamide)
- la N-chlorosuccinide
- la trichloromélamine
- la chloroazodine

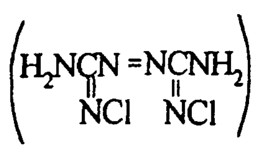

- les dérivés N-chloro des acides cyanuriques, de préférence l'acide trichloroisocyanurique et/ou le sodium dichloroisocyanurique dihydrate,
- les N-chlorohydantoïnes, de préférence la 1-bromo-3-chloro-5,5'-diméthyldantoïne, ou la 1,3-dichloro-5,5-idiméthylhydantoine,
- et leurs mélanges.

Ce groupe d'antiseptiques correspond sensiblement à la famille des N-chloramines qui comprend les dérivés des amines dans lesquelles une ou deux des valences de l'azote trivalent sont substituées par du chlore. En présence d'eau, les N-chloramines produisent de l'acide hypochloreux HClO ou des sels de cet acide tels que NaClO. HClO est NaClO sont des dérivés chlorés actifs, doués d'une grande capacité bactéricide, que l'on exploite dans le cadre du système selon l'invention.

Selon une caractéristique avantageuse de l'invention le système qu'elle concerne est obtenu par mélange de la composition POS (I) avec l'agent biocide sous forme de suspension ou de solution de préférence alcoolique.

L'agent biocide (II) est associé à au moins un auxiliaire antiseptique (III) différent des antiseptiques fonctionnant par libération de chlore et de préférence choisi dans le groupe des formulations tensioactives comportant un ou plusieurs ammoniums quaternaires et éventuellement au moins un activateur séquestrant, de préférence sélectionné parmi les complexants d'ions métalliques.

Selon un autre mode de définition de l'agent biocide (II), il peut être précisé que celui-ci est avantageusement exempt d'activité inhibitrice sur la réticulation de la composition POS (I).
En pratique, le système selon l'invention est tel que :

- la composition POS (I) comprend :
    - ($I_a$) : au moins une huile diorganopolysiloxane bloquée à chaque extrémité de sa chaîne par un motif vinyldiorganosiloxyle dont les radicaux organiques liés aux atomes de silicium sont choisis parmi les radicaux méthyle, éthyle et phényle, au moins 60% molaire de ces radicaux étant des radicaux méthyle, de viscosité 100 à 500 000, de préférence de 1 000 à 200 000 mPa.s à 25°C;
    - ($I_b$) : au moins un organohydrogénopolysiloxane choisi parmi les homopolymères et copolymères liquides linéaires ou en réseau présentant par molécule au moins 3 atomes d'hydrogène liés à des atomes de silicium différents et dont les radicaux organiques liés aux atomes de silicium sont choisis parmi les radicaux méthyle, éthyle et 60% au moins de ces radicaux étant des radicaux méthyle, le produit ($I_b$) étant utilisé en quantité telle que le rapport molaire des fonctions hydrure sur les groupes vinyle soit compris entre 1,1 et 4:
    - ($I_c$) : une quantité catalytiquement efficace d'un catalyseur au platine.
    - ($I_d$) : un ou plusieurs additifs fonctionnels choisis de préférence dans le groupe comprenant les silices, les alumines, les silicates, les disiloxanes vinyles, les huiles de vaseline, les paraffines et leurs mélanges.

- l'agent biocide (II) comporte une chloramine de préférence la tosylchloramide sodique ou l'un de ses analogues chlorés,
- l'agent biocide (II) est associé à un auxiliaire antiseptique (III), de préférence le chlorure de benzalkonium, avantageusement complété par un activateur-séquestrant, de préférence de l'EDTA.

Selon un autre de ses aspects, l'invention vise également un matériau pour la prise d'empreintes, notamment dentaires, comprenant un système tel que défini ci-dessus.
Avantageusement, ce matériau comporte en outre des adjuvants sélectionnés parmi les édulcorants, de préférence les édulcorants de synthèse, et/ou parmi les arômes, et/ou parmi les les colorants et/ou parmi les anti-inflammatoires, et/ou parmi les produits isotaniques, de préférence les saccharides, plus préférentiellement encore les saccharides hydrogénés, le sorbitol étant tout spécialement retenu, et leurs mélanges.
A titre d'exemple d'édulcorants de synthèse, on peut citer les produits commercialisés sous les marques ASPARTAM®, ACESULFAM®....
L'arôme de menthe fait partie des arômes susceptibles d'être mis en oeuvre.
Comme exemple d'adjuvant anti-inflammatoire, on peut citer l'allantoïne ou l'un de ses analogues.
Le chlorure de benzalkonium ou l'un de ses analogues est un ammonium quaternaire tensioactif et antiseptique, disponible dans le commerce.
L'agent biocide (II) mis en oeuvre dans le cadre de l'invention est une formulation comprenant une chloramine ainsi que des auxiliaires antiseptiques (III), avec éventuellement un activateur séquestrant et des adjuvants. Le calbénium de composition suivante est un exemple de ces formulations :

- EDTA (Acide Ethylène Diamine Tétraacétique)
  ou l'un de ses analogues 100 ± 50 parties en poids
- Tosylchloramide sodique ou l'un de ses analogues 2 ± 1 partie en poids
- Chlorure de benzalkonium ou l'un de ses analogues 12 ± 6 parties en poids
- Aspartam et tout édulcorant synthétique ou hémi-synthétique 8 ± 4 parties en poids
- Menthe ou tout arôme 10 ± 5 parties en poids
- Allantoïne ou l'un de ses analogues 6 ± 3 parties en poids
- Sorbitol ou l'un de ses analogues 132 ± 66 parties en poids

formulation pour une dose de 2,5 g nécessaire pour un litre d'eau.
La présente invention concerne également un procédé de préparation du système ou du matériau tel que décrit ci-dessus. Ce procédé est caractérisé en ce qu'il consiste essentiellement à mélanger les composés du POS (I), l'agent biocide (II) et l'auxiliaire antiseptique (III) avantageusement complété par l'activateur-séquestrant, et éventuellement les adjuvants tels que mentionnés ci-dessus.
Ce mélange s'effectue de manière traditionnelle par les moyens techniques appropriés et connus de l'homme du métier.
Selon une proposition avantageuse évoquée ci-dessus, il est préférable d'introduire l'agent biocide II sous forme d'une solution alcoolique.

La présente invention a également pour objet l'utilisation du système ou du matériau, tel que décrit ci-dessus, pour la prise d'empreintes dentaires, cette utilisation étant caractérisée en ce qu'elle consiste essentiellement à faire en sorte que la réticulation de l'élastomère silicone s'initie, de préférence, par mélange des composants A et B, à prendre l'empreinte dentaire et à laisser se poursuivre la réticulation jusqu'à ce que l'élastomère ait suffisamment réticulé et soit suffisamment dur.

L'invention sera mieux comprise à l'aide des exemples qui suivent et qui décrivent la préparation du système élastomère silicone biocide selon l'invention ainsi que son évaluation en termes de propriétés mécaniques et de propriétés antiseptiques.

**EXEMPLES**

**EXEMPLES 1 A 3 ET ESSAIS 1 A 3**

I - Dans les exemples 1 à 3 on prépare les bicomposants réticulant par des réactions de polyaddition, conduisant à des produits pâteux de première empreinte (bicomposants dits de type I).

**1. Liste des matières premières utilisées :**

**<u>1.1 - Partie A du bicomposant</u>**

- POS ($I_a$) : Huile polydiméthylsiloxane bloquée à chacune des extrémités des chaînes par un motif $(CH^3)_2$ $ViSiO_{0,5}$ de viscosité 100 000 mPa.s et contenant environ 0,0024 fonction vinyle (Vi) dans 100 g d'huile, soit environ 0,065% en poids de fonction Vi;
- POS ($I_{a1}$) : Huile de même nature que POS ($I_a$) mais ayant une viscosité de 600 mPa.s, contenant environ 0,014 fonction Vi dans 100 g d'huile, soit environ 0,38% en poids de fonction Vi;
- POS ($I_{a2}$) : ralentisseur de la réaction de polyaddition consistant dans du divinyltétraméthyldisiloxane;

*Additifs fonctionnels ($I_d$)*

- *Charge 1* : silicate d'aluminium et de sodium, commercialisé sous la dénomination SIPERNAT 44;
- *Charge 2* : alumine hydratée, commercialisée sous la dénomination ALLUMINA M 15/B;
- *Charge 3* : matière siliceuse à base de terres diatomées, commercialisée sous la dénomination DICALITE WHITE FILLER;
- *Ingrédient 1* : huile de vaseline;
- *Ingrédient 2* : paraffine, commercialisée sous la dénomination PARAFFINE RAFF. LASTRE 52/5;
- Catalyseur ($I_c$): platine zéro complexé par du divinyltétraméthyldisiloxane : on engage une solution dans le divinyltétraméthyldisiloxane d'un complexe de platine à environ 11% en poids de platine zéro ligandé par du divinyltétraméthyldisiloxane; les quantités de ce catalyseur sont exprimées en parties en poids de solution mise en oeuvre.

**<u>1.2. - Partie B du bicomposant :</u>**

- POS ($I_a$) : cf. partie A;
- POS ($I_{a1}$) : cf. partie A;
- POS ($I_b$) : gomme poly(diméthyl)(méthylvinyl)siloxane bloquée à chacune des extrémités des chaînes par un motif $(CH_3)_2ViSiO_{0,5}$ ayant une masse moléculaire moyenne en poids de 540 000 g/mole, contenant environ 0,00185 fonction Vi dans 100 g de gomme, soit environ 0,05% en poids de fonction Vi;
- POS ($I_{b1}$) : huile poly(diméthyl)(hydrogénométhyl)siloxane bloquée à chacune des extrémités des chaînes par un motif $(CH_3)_2HiSiO_{0,5,}$ ayant une viscosité de 30 mPa.s. et contenant environ 0,25 fonction H dans 100 g d'huile, soit environ 0,25% en poids de H;

*Additifs fonctionnels ($I_d$)*

- *Charge 1* : cf. partie A;
- *Charge 2* : cf. partie A;
- *Charge 3* : cf. partie A;
- *Ingrédient 1* : cf. partie A;
- *Ingrédient 2* : cf. partie A;
- Bactéricide (II): solution à 80% en poids de calbénium (80 parties) dans l'alcool éthylique à 96° (20 parties).

**2. Constitution des parties A et B des bicomposants I testés :**

| | Partie A | Partie B | | |
|---|---|---|---|---|
| POS ($I_a$) | 14,45 * | 11,4 | | |
| POS II ($I_{a1}$) | 7,40 | 4,6 | | |
| POS III ($I_{a2}$) | 0,035 | - | | |
| POS IV ($I_b$) | - | 4,8 | | |
| POS V ($I_{b1}$) | - | 2,45 | | |
| *Additifs fonctionnels ($I_d$)* | | | | |
| *Charge 1* | 40,0 | 31,2 | | |
| *Charge 2* | 21,0 | 29,6 | | |
| *Charge 3* | 10,0 | 8,25 | | |
| *Ingrédient 1* | 6,5 | 7,0 | | |
| *Ingrédient 2* | 0,6 | 0,7 | | |
| Catalyseur ($I_c$) | 0,015 | - | | |
| Bactéricide (II) | - | Ex. 1 | Ex. 2 | Ex. 3 |
| | | 0,25 | 0,75 | 1,25 |

(* : parties en poids)

**3. Préparation des compositions**

**3.1 - Partie A :**

**(1)** Dans un mélangeur planétaire, on introduit à 23°C les constituants suivants : POS ($I_a$), POS ($I_{a1}$), charge 1 et charge 2; l'ensemble est homogénéisé par agitation à 20 tours/min pendant 1 heure.
**(2)** L'agitation est ensuite arrêtée et on ajoute alors la charge 3 : nouvelle homogénéisation à 20 tours/min pendant 1 heure.
**(3)** L'agitation est ensuite arrêtée et on ajoute alors les ingrédients 1 et 2 : nouvelle homogénéisation à 20 tours/min pendant 1 heure.
**(4)** L'agitation est ensuite arrêtée et on ajoute alors le catalyseur et le POS ($I_{a2}$) : nouvelle homogénéisation à 20 tours/min pendant 30 minutes.
**(5)** Puis, sans arrêter l'agitation, on procède au dégazage de la masse en opérant à 23°C, sous une pression réduite de $226.10^2$ Pa, pendant 20 minutes.

**3.2 - Partie B :**

Dans le mélangeur précédent, on réalise les étapes suivantes :

**(1)** Introduction des POS ($I_a$), POS ($I_{a1}$), POS ($I_b$), charge 1 et charge 2 et homogénéisation à 23°C, sous une agitation de 20 trs/min, pendant 1 heure;
**(2)** Arrêt agitation et ajout de la charge 3 : homogénéisation 3 heures à 20 trs/min;
**(3)** Arrêt agitation et ajout des ingrédients 1 et 2 : homogénéisation 1 heure à 20 trs/min;
**(4)** : Arrêt agitation et ajout du bactéricide en quantité voulue et du POS ($I_{b1}$): homogénéisation 30 minutes à 20 trs/min;
**(5)** : Dégazage comme indiqué ci-avant pour la partie A;

**3.3. Bicomposants A + B de type I :**

La composition RTV dentaire est obtenue par mélange, à température ambiante (23°C), de 50 parties en poids de la partie A et de 50 parties en poids de la partie B. La réticulation de chaque composition bicomposante s'effectue à température ambiante après réalisation du mélange.

**II -** Des essais comparatifs (essais 1 à 3) ont été réalisés mettant en oeuvre des bicomposants de nature semblable, mais dans lesquels le bactéricide consiste dans du chlorure de benzalkonium (bicomposants dits de type II).

**1. Liste des matières premières utilisées :**

**1.1. Partie A :**

- POS ($I_a$), POS ($I_{a1}$), POS ($I_{a2}$) et charge 1 : cf. partie A, dans § I.1.1;
- Charge 2 : alumine hydratée, commercialisée sous la dénomination ALLUMINE ALCOA M 10;
- Charge 3 : matière siliceuse à base de terres diatomées, commercialisée sour la dénomination CELITE SUPER FLOS;
- Ingrédients 1 et 2 et catalyseur : cf. partie A, dans § I-1.1.

**1.2. Partie B :**

- POS ($I_a$), POS ($I_{a1}$), POS ($I_b$), POS ($I_{b1}$) et charge 1 : cf. partie B, dans § I-1.2.;
- Charges 2 et 3 : cf. partie A, dans § II-1.1.;
- Ingrédients 1 et 2 : cf. partie B, dans § I-1.2.;
- Bactéricide (II): solution à 50% en poids de chlorure de benzalkonium (50 parties) dans l'alcool éthylique à 96° (50 parties)

**2. Constitution des parties A et B des bicomposants II testés :**

| | Partie A | Partie B |
|---|---|---|
| POS ($I_a$) | 14,45 * | 11,3 |
| POS ($I_{a1}$) | 7,40 | 4,0 |
| POS ($I_{a2}$) | 0,035 | - |
| POS ($I_b$) | - | 4,8 |
| POS ($I_{b1}$) | - | 3,1 |

(* : parties en poids)

(suite)

| | Partie A | Partie B | | |
|---|---|---|---|---|
| *Additifs fonctionnels ($I_d$)* | | | | |
| *Charge 1* | 40,0 | 30,8 | | |
| *Charge 2* | 21,0 | 28,8 | | |
| *Charge 3* | 10,0 | 8,9 | | |
| *Ingrédient 1* | 6,5 | 7,0 | | |
| *Ingrédient 2* | 0,6 | 0,7 | | |
| Catalyseur($_{Ic}$) | 0,015 | - | | |
| Bactéricide (II) | - | Essai 1 | Essai 2 | Essai 3 |
| | | 0,5 | 1,0 | 2,0 |

**3. Préparations des compositions :**

**3.1 Partie A :**

On procède comme ci-avant dans le § I-3.1..

**3.2. Partie B :**

On procède comme indiqué ci-avant dans le § I-3.2., mais aves les légères modifications suivantes :

- étape (1) à (5) : agitation à 50 tours/min;
- étape (3) : ajout des ingrédient 1 et 2 et du bactéricide;
- étape (4) : ajout du seul POS V et homogénéisation 1 heure.

**3.3.Bicomposants A + B de type II :**

La réticulation des bicomposant s'effectue, comme indiqué ci-avant dans le § I-3.3., à température ambiante après mélange des 2 parties A et B dans un rapport 50/50 en poids.

**III - Résultats**

Ils sont rassemblés dans le tableau 1 suivant. Ce tableau indique les teneurs en agent bactéricide qui ont été introduites selon la technique décrite précédemment, ainsi que les temps de travail et temps de prise des produits obtenus. Le temps de travail correspond au temps pendant lequel le mélanges des 2 parties A et B conserve un comportement fluide; au-delà, le matériau acquière les caractéristiques d'un élastomère. Le temps de prise correspond au temps nécessaire pour que l'empreinte dentaire devienne manipulable.

**Tableau 1**

| | Ex. 1 | Ex. 2 | Ex. 3 | Essai 1 | Esai 2 | Essai 3 |
|---|---|---|---|---|---|---|
| Type bicomposant | I | I | I | II | II | II |
| Bactéricide<br>• nature :<br>• % en poids dans A + b | Calbénium<br>0,1 | calbénium<br>0,3 | calbénium<br>0,5 | chlorure de benzalkonium<br>0,125 | chlorure de benzalkonium<br>0,25 | chlorure de benzalkonium<br>0,5 |
| Propriétés initiales mesurées après préparation des parties A et B et leurs mélanges :<br>• temps de travail :<br>• temps de prise | 1 min 50 s<br>2 min 20 s | 1 min 50 s<br>2 min 30 s | 2 min<br>2 min 50 s | 2 min<br>5 min | 2 min 15 s<br>6 min | 2 min 30 s<br>6 min 30 s |
| Propriétés mesurées après stockage des parties A et B séparées x jours à 23°C :<br>• temps de travail :<br>• temps de prise : | x = 180<br>2 min<br>2 min 30 s | x = 180<br>2 min 50 s<br>2 min 50 s | x = 180<br>2 min 10 s<br>3 min | | | x = 30<br>-<br>>10 min |

EXEMPLES 4 A 6 ET ESSAIS 4 A 6 :

I - Dans les exemples 4 à 6, on prépare des bicomposants réticulant par des réactions de polyaddition, conduisant à des produits fluides de deuxième empreinte (bicomposants dits de type III).

**1. Liste des matières premières utilisées :**

**1.1 - Partie A :**

- POS ($I_a$) et ($I_{a1}$) : cf. partie A, exemples 1-3, § I-1.1.;

*Additifs fonctionnels ($I_d$)*

- *Charge 4 :* silice de combustion, commercialisée sous la dénomination AEROSIL 200, traitée avec de l'hexaméthyldisilazane;
- *Charge 5 :* quartz broyé de diamètre particulaire moyen de 10 µm, commercialisé sous la dénomination SICRON SA 600;
- *Charge 6 :* $TiO_2$;
- *Ingrédient 3* : alcool gras en $C_8$-$C_{10}$ polyoxyéthylylé et propylé, commercialisé sous la dénomination SOPROFOR BO 327;
- Catalyseur ($I_c$): cf. partie A, exemples 1-3, § I-1.1.

**1.2. - Partie B:**

- POS ($I_a$) et ($I_{a1}$): cf. partie A, juste ci-avant;
- POS ($I_{b1}$): cf. partie B, exemple 1-3, § I-1.2.;

*Additifs fonctionnels ($I_d$)*

- *Charges 4, 5 et 6* : cf. partie A, juste ci-avant;
- *Ingrédient 3* : cf. partie A, juste ci-avant;
- Bactéricide : solution à 80% en poids de calbénium (80 parties) dans l'alcool éthylique à 96° (20 parties).

**2. Constitution des parties A et B des bicomposants III testés :**

| | Partie A | Partie B |
|---|---|---|
| POS ($I_a$) | 2,0 * | 6,0 |
| POS ($I_{a1}$) | 52,0 | 42,3 |
| POS ($I_{b1}$) | - | 8,0 |
| *Additifs fonctionnels ($I_d$)* | | |
| *Charge 4* | 12,0 | 12,0 |
| *Charge 5* | 34,5 | 35,0 |
| *Charge 6* | 0,6 | 0,5 |

(* : parties en poids)

(suite)

| | Partie A | Partie B | | |
|---|---|---|---|---|
| *Additifs fonctionnels ($I_d$)* | | | | |
| *Ingrédient 3* | 0,3 | 0,3 | | |
| Catalyseur ($I_c$) | 0,01 | - | | |
| Bactéricide (II) | - | Ex. 4 | Ex. 5 | Ex. 6 |
| | | 0,25 | 0,75 | 1,25 |

3. Préparation des compositions

**3.1 - Partie A :**

**(1)** Dans un premier temps, on prépare un mélange de base (en abréviation : MB) par malaxage à température ambiante (23°C) de 24 parties en poids de charge 4 et de 56 parties en poids de POS (II).
**(2)** Pour la préparation de la partie A, on mélange dans un disperseur à turbine 40 parties du MB avec le POS I, le reliquat de POS (II) (24 parties), les charges 5 et 6 et l'ingrédient 3 : on opère à 23°C, sous une agitation de 450 trs/min, pendant 2 heures.
**(3)** L'agitation est ensuite arrêtée puis on vérifie que la température de la masse est ≤ à 70°C avant d'ajouter le catalyseur : homogénéisation 10 minutes à 450 trs/min, puis dégazage de la masse en opérant sous une pression réduite de $266.10^2$ Pa pendant 10 minutes.

**3.2 - Partie B :**

**(1)** On mélange 40 parties du reste MB avec le POS ($I_a$), le reliquat de POS ($I_a$), le reliquat de POS ($I_{a1}$) (14,3 parties), les charges 5 et 6 et l'ingrédient 3 : ensuite, homogénéisation 1 heure à 450 trs/min.
**(2)** L'agitation est ensuite arrêtée et on ajoute alors le POS ($I_{b1}$) : on reprend alors homogénéisation 10 minutes à 450 trs/min.
**(3)** : Nouvel arrêt de l'agitation et, après avoir vérifié que la température de la masse est ≤ à 70°C, on ajoute le bactéricide : ensuite, homogénéisation 10 minutes à 450 trs/min; puis dégazage comme indiqué ci-avant, en fin de la partie A.

**3.3. Bicomposants A + B de type III :**

La réticulation des bicomposants s'effectue là aussi à température ambiante (23°C) après mélange des 2 parties A et B dans un rapport 50/50 en poids.

**II -** Des essais comparatifs (essais 4 à 6) ont été réalisés mettant en oeuvre des bicomposants de nature semblable, mais dans lesquels le bactéricide consiste dans du chlorure de benzalkonium (bicomposants dits de type VI).

**1. Liste des matières premières utilisées :**

**1.1. Partie A :**

- POS ($I_a$) et ($Ia_1$): cf. partie A, exemples 4-6, § I-1.1.;
- POS ($Ia_2$): cf. partie A, exemples 1-3, § I-1.1.;

*Additifs fonctionnels ($I_d$)*

- *Charges 4, 5 et 6* : cf. partie A, exemples 4-6, § I-1.1.;
- *Charge 7* : silice de combustion, commercialisée sous la dénomination AEROSIL 200 traitée avec de l'octaméthylcyclotétrasiloxane;
- Ingrédient 3 et catalyseur ($I_c$): cf. partie A, exemples 4-6, § I-1.1.

**1.2. Partie B :**

- POS ($I_a$) et ($I_{b2}$) + charges 4 et 5 + ingrédient 3 : cf. partie B, exemples 4-6, § I-1.2.;
- Bactéricide : solution à 50% en poids de chlorure de benzalkonium (50 parties) dans l'alcool éthylique à 96° (50 parties)

**2. Constitution des parties A et B des bicomposants IV testés :**

| | Partie A | Partie B | | |
|---|---|---|---|---|
| POS ($I_a$) | 2,0* | - | | |
| POS ($I_{a1}$) | 49,46 | 40,7 | | |
| POS ($I_{a2}$) | 0,03 | - | | |
| POS ($I_{b2}$) | - | 11,0 | | |
| *Additifs fonctionnels ($I_d$)* | | | | |
| *Charge 4* | 12,4 | 14,6 | | |
| *Charge 5* | 34,5 | 34,0 | | |
| *Charge 6* | 0,5 | - | | |
| *Charge 7* | 1,0 | - | | |
| *Ingrédient 3* | 0,1 | 0,3 | | |
| Catalyseur ($I_c$) | 0,01 | - | | |
| Bactéricide (II) | - | Essai 5 | Essai 6 | Essai 7 |
| | | 0,5 | 1,0 | 2,0 |

(* : parties en poids)

**3. Préparation des compositions :**

**3.2. Partie A :**

On procède comme indiqué ci-avant dans le § I-3.1. des exemples 4 à 6, mais avec les modifications suivantes :

étape (1) : le MB est préparé par malaxage de 27 parties en poids de charge 4 et de 63 parties en poids de POS ($I_{a1}$);
étape (2) : on mélange 41,3 parties du MB avec le POS ($I_a$), le reliquat de POS ($I_{a1}$) (20,55 parties), les charges 5, 6 et 7 et l'ingrédient 3;
étape (3) : on ajoute le catalyseur et le POS ($I_{a2}$).

**3.2. Partie B**

On procède comme indiqué ci-avant dans le § I-3.2. des exemples 4 à 6, mais avec les modifications

suivantes :

- étape (1) : on mélange 48,7 parties du reste du MB avec le reliquat de POS ($I_{a1}$) (6,61 parties);
- étape (2) : on lui ajoute la charge 5 à la place du POS ($I_{b1}$) et on homogénéise pendant 2 heures;
- étape (3) : on ajoute cette fois, à côté du bactéricide, le POS ($I_{b1}$) et l'ingrédient 3, et on homogénéise pendant 1 heure 30 minutes.

**3.2. Bicomposants A + B du type IV**

La réticulation des bicomposants s'effectue là encore à température ambiante (23°C) après mélange des 2 parties A et B dans un rapport 50/50 en poids.

**III - Résultats**

Ils sont rassemblés dans le tableau 2 suivant.

**Tableau 2**

| | Ex. 4 | Ex. 5 | Ex. 6 | Essai 4 | Esai 5 | Essai 6 |
|---|---|---|---|---|---|---|
| Type bicomposant | III | III | III | IV | IV | IV |
| Bactéricide<br>• nature :<br>• % en poids dans A + B | calbénium<br>0,1 | calbénium<br>0,3 | calbénium<br>0,5 | chlorure de benzalkonium<br>0,125 | chlorure de benzalkonium<br>0,25 | chlorure de benzalkonium<br>0,5 |
| Propriétés initiales mesurées après préparation des parties A et B et leurs mélanges :<br>• temps de travail :<br>• temps de prise | 2 min 30 s<br>5 min 10 s | 2 min 40 s<br>6 min | 2 min 50 s<br>8 min | 2 min<br>5 min | 3 min<br>8 min | 4 min<br>>15min |
| Propriétés mesurées après stockage des parties A et B séparées x jours à 23°C :<br>• temps de travail :<br>• temps de prise : | x = 180<br>2 min 30 s<br>5 min 20 s | x = 180<br>2 min 50 s<br>6 min 15 s | x = 180<br>2 min 55 s<br>8 min. 20 s | | | x = 30<br>-<br>>30 min |

EXEMPLE 7 - ACTIVITE BACTERICIDE

Les activités bactéricides des systèmes selon les exemples 1 à 6 et selon les essais 1 à 6 ont été évalués de manière suivante.

On réalise des films de 2 mm d'épaisseur et de 2,5 cm de côté, par réticulation à température ambiante de mélange approprié de parties A et B des systèmes testés.

Les carrés de silicones sont mis à tremper dans une suspension bactérienne de *Staphylococcus aureus* titrant 105 CFU/ml, de façon à ce que les germes soient en contact aussi bien avec la tranche qu'avec la surface des carrés.

Après un temps de contact de 30 mn, les films sont retirés et maintenus en condition d'humidité relative (TPS 50), et ceci jusqu'à la mise en culture, soit après 6 h, 24 h, 3 jours et une semaine, et à température du laboratoire.

La culture se fera par inclusion en gélose et en milieu Trypticas-soja à 37 ± 1°C pendant 24 à 48 heures

Avant l'incubation, les géloses sont ensemencées avec une culture de *Staphylococcus aureus* ($8.10^4$ CFU) , cette souche ayant été choisie comme germe représentatif du milieu buccal.

On utilise des témoins exempts de biocides élastomères silicones de même type que des systèmes selon les exemples 1 à 6.

Résultats

| | Témoin | Systèmes silicones biocide ex. 1 à 6 |
|---|---|---|
| Culture après 6 h de contact | Nombreuses colonies (environ 300 CFU/boîte) | Quelques colonies subsistent (environ 30 CFU/boîte) |
| Culture après 24 h de contact | Pousse sur les silicones (environ 100 CFU /boîte) | Tout négatif |
| Culture après 3 jours de contact | Pousse sur les silicones (environ 36 CFU /boîte) | Tout négatif |
| Culture après 7 jours de contact | | Tout négatif |

## Revendications

**1.** Système élastomère silicone, utilisable notamment pour la prise d'empreintes, ayant des propriétés biocides et **caractérisé en ce que**

- la composition POS (I) comprend :
  - ($I_a$) : au moins une huile diorganopolysiloxane bloquée à chaque extrémité de sa chaîne par un motif vinyldiorganosiloxyle dont les radicaux organiques liés aux atomes de silicium sont choisis parmi les radicaux méthyle, éthyle et phényle, au moins 60% molaire de ces radicaux étant des radicaux méthyle, de viscosité 100 à 500 000, de préférence de 1 000 à 200 000 mPa.s à 25°C;
  - ($I_b$) : au moins un organohydrogénopolysiloxane choisi parmi les homopolymères et copolymères liquides linéaires ou en réseau présentant par molécule au moins 3 atomes d'hydrogène liés à des atomes de silicium différents et dont les radicaux organiques liés aux atomes de silicium sont choisis parmi les radicaux méthyle, éthyle et 60% au moins de ces radicaux étant des radicaux méthyle, le produit ($I_b$) étant utilisé en quantité telle que le rapport molaire des fonctions hydrure sur les groupes vinyle soit compris entre 1,1 et 4:
  - ($I_c$) : une quantité catalytiquement efficace d'un catalyseur au platine.
- \- ($I_d$) : un ou plusieurs additifs fonctionnels choisis de préférence dans le groupe comprenant les silices, les alumines, les silicates, les disiloxanes vinyles, les huiles de vaseline, les paraffines et leurs mélanges.
- l'agent biocide (II) comporte une chloramine de préférence la tosylchloramide sodique ou l'un de ses analogues chlorés,
- l'agent biocide (II) est associé à un auxiliaire antiseptique (III), de préférence le chlorure de benzalkonium, avantageusement complété par un activateur-séquestrant, de préférence de l'EDTA.

**2.** Système élastomère silicone selon la revendication 1 **caractérisé en ce que** l'agent biocide II est choisi dans le groupe de précurseur de chlore actif comprenant :

- la chloramine B (sodium-N-chlorobenzène sulfonamide),
- la dichloroamine T (N,N-dichloro-p-toluène sulfonamide),
- la N-trichlorométhylmercapto-4-cyclohexène-1,2-dicarboxylamide),
- l'halazone (acide benzoïque p-n-dichlorosulfonamide)
- la N-chlorosuccinide
- la trichloromélamine
- la chloroazodine

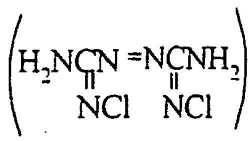

- les dérivés N-chloro des acides cyanuriques, de préférence l'acide trichloroisocyanurique et/ou le sodium dichloroisocyanurique dihydrate,
- les N-chlorohydantoïnes, de préférence la 1-bromo-3-chloro-5,5'-diméthyldantoïne, ou la 1,3-dichloro-5,5'-diméthylhydantoine,
- et leurs mélanges.

**3.** Système élastomère silicone selon l'une quelconque des revendications précédentes, **caractérisé en ce que** la composition POS (I) est une composition bi-composante A-B, précurseur de l'élastomère réticulé à température ambiante ou à la chaleur, par mélange de composants A et B, isolément non durcissables.

**4.** Système élastomère silicone selon l'une quelconque des revendications précédentes caractérisé en ce l'agent biocide (II) est exempt d'activité inhibitrice sur la réticulation de la compostion POS (I).

**5.** Matériau pour la prise en charge d'empreinte, notamment dentaire, **caractérisé en ce qu'**il comprend un système selon l'une quelconque des revendications 1 à 4.

**6.** Matériau selon la revendication 5, **caractérisé en ce qu'**il comporte des adjuvants sélectionnés parmi les édulcorants, de préférence parmi les édulcorants de synthèse, et/ou parmi les arômes, et/ou parmi les colorants et/ou parmi les anti-inflammatoires, et/ou parmi les produits isotoniques, de préférence les saccharides, et plus préférentiellement encore les saccharides hydrogénés, le sorbitol étant tout spécialement retenu, et leurs mélanges.

**7.** Procédé de préparation du système selon l'une quelconque des revendications 1 à 4 et/ou du matériau selon la revendication 5 ou la revendication 6, **caractérisé en ce qu'**il consiste esentiellement à mélanger les composés du POS (I), l'agent biocide (I) et l'auxiliaire (III) avantageusement complété par l'activateur-séquestrant, et éventuellement les adjuvants tels que définis dans la revendication 6.

**8.** Utilisation du système selon l'une des revendications 1 à 4 et/ou du matériau selon la revendication 5 ou la revendication 6, pour la prise d'empreinte, notamment dentaire, **caractérisée en ce qu'**elle consiste essentiellement à faire en sorte que la réticulation de l'élastomère silicone s'initie, de préférence par mélange des composants A et B, à prendre l'empreinte dentaire et à laisser se poursuivre la réticulation jusqu'à ce que l'élastomère ait suffisamment réticulé et soit suffisamment dur.

## Claims

**1.** Silicone elastomer system, which can be used in particular for taking impressions, having biocidal properties and **characterized in that**

- the POS composition (I) comprises:

  - ($I_a$): at least one diorganopolysiloxane oil blocked at each end of its chain by a vinyldiorganosiloxyl unit in which the organic radicals linked to the silicon atoms are chosen from methyl, ethyl and phenyl radicals, at least 60 mol% of these radicals being methyl radicals, having a viscosity of 100 to 500 000,

preferably of 1 000 to 200 000 mPa.s at 25°C;

- ($I_b$): at least one organohydrogenopolysiloxane chosen from the linear or lattice liquid copolymers and homopolymers having per molecule at least three hydrogen atoms linked to different silicon atoms and in which the organic radicals linked to the silicon atoms are chosen from methyl and ethyl radicals and at least 60% of these radicals being methyl radicals, the product ($I_b$) being used in a quantity such that the molar ratio of the hydride functions to the vinyl groups is between 1.1 and 4:
- ($I_c$) : a catalytically effective quantity of a platinum catalyst.

- - ($I_d$): one or more functional additives preferably chosen from the group comprising silicas, aluminas, silicates, vinyl disiloxanes, liquid paraffins, paraffins and mixtures thereof.
- the biocidal agent (II) comprises a chloramine, preferably sodium tosylchloramide or one of its chlorinated analogues,
- the biocidal agent (II) is optionally combined with an antiseptic auxiliary agent (III), preferably benzalkonium chloride, advantageously supplemented with an activator-sequestrant, preferably EDTA.

**2.** Silicone elastomer system according to claim 1, **characterized in that** the biocidal agent II is chosen from the active chlorine precursor group comprising:

- chloramine B (sodium N-chlorobenzenesulphonamide),
- dichloramine T (N,N-dichloro-p-toluenesulphonamide),
- N-trichloromethylmercapto-4-cyclohexene-1,2-dicarboxylamide),
- halazone (p-n-dichlorosulphonamidebenzoic acid)
- N-chlorosuccinide
- trichloromelamine
- chloroazodin

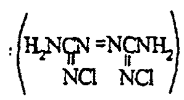

- N-chloro derivatives of cyanuric acids, preferably trichloroisocyanuric acid and/or sodium dichloroisocyanuric dihydrate,
- N-chlorohydantoins, preferably 1-bromo-3-chloro-5,5'-dimethyldantoin, or 1,3-dichloro-5,5'-dimethylhydantoin,
- and mixtures thereof.

**3.** Silicone elastomer system according to either of the preceding claims, **characterized in that** the POS composition (I) is a bicomponent composition A-B, a precursor of the elastomer crosslinked at room temperature or by heat, by mixing components A and B, which are not hardenable separately.

**4.** Silicone elastomer system according to any one of the preceding claims, **characterized in that** the biocidal agent (II) is free of inhibitory activity on the crosslinking of the POS composition (I).

**5.** Material for taking impression, in particular dental impression, **characterized in that** it comprises a system according to any one of claims 1 to 4.

**6.** Material according to claim 5, **characterized in that** it comprises adjuvants selected from sweeteners, preferably synthetic sweeteners, and/or from flavourings, and/or from colourings and/or from anti-inflammatory agents, and/or from isotonic products, preferably saccharides, more preferably still hydrogenated saccharides, sorbitol being most especially selected, and mixtures thereof.

**7.** Process for preparing the system according to any one of claims 1 to 4 and/or of the material according to claim 5 or claim 6, **characterized in that** it essentially consists in mixing the POS compounds (I), the biocidal agent (I) and the auxiliary agent (III) advantageously supplemented with the activator-sequestrant, and optionally the adjuvants as defined in claim 6.

**8.** Use of the system according to one of claims 1 to 4 and/or of the material according to claim 5 or claim 6, for taking

impression, in particular dental impression, **characterized in that** it essentially consists in ensuring that the crosslinking of the silicone elastomer is initiated, preferably by mixing components A and B, in taking the dental impression and in allowing the crosslinking to continue until the elastomer is sufficiently crosslinked or is sufficiently hard.

**Patentansprüche**

1. Siliconelastomer-System mit bioziden Eigenschaften, insbesondere verwendbar für das Abformen, **dadurch gekennzeichnet, daß**

   - die Verbindung POS (I) umfaßt:

     - ($I_a$) : mindestens ein Diorganopolysiloxan-Öl, blockiert an jedem Ende seiner Kette durch eine Struktureinheit Vinyldiorganosiloxyl, deren an Siliciumatome gebundene organische Reste unter den Resten Methyl, Ethyl und Phenyl ausgewählt werden, wobei mindestens 60 Mol-% dieser Reste Methylreste sind, und wobei das Öl eine Viskosität von 100 bis 500.000, vorzugsweise von 1.000 bis 200.000 mPa.s bei 25 °C besitzt;
     - ($I_b$) : mindestens ein Organohydrogenopolysiloxan, ausgewählt unter den linearen flüssigen oder vernetzten Homopolymeren und Copolymeren, die pro Molekül mindestens drei an unterschiedliche Siliciumatom gebundene Wasserstoffe aufweisen und deren an Siliciumatome gebundene organische Reste unter den Resten Methyl und Ethyl ausgewählt werden und mindestens 60 % dieser Reste Methylreste sind, wobei das Produkt ($I_b$) in einer solchen Menge verwendet wird, daß das molare Verhältnis der Hydridfunktionen zu den Vinylgruppen zwischen 1,1 und 4 beträgt;
     - ($I_c$) : eine katalytisch wirksame Menge eines Platinkatalysators,

   - - ($I_d$) : einen oder mehrere funktionelle Zusatzstoffe, vorzugsweise aus der Gruppe gewählt, die Kieselerden, Tonerden, Silikate, Vinyl-Disiloxane, Vaselineöle, Paraffine und ihre Mischungen umfaßt,
   - das biozide Mittel (II) umfaßt ein Chloramin, vorzugsweise Natrium-tosylchloramid oder eines seiner chlorierten Analogen,
   - das biozide Mittel (II) ist mit einem antiseptischen Hilfsmittel (III) assoziiert, vorzugsweise Benzalkoniumchlorid, vorteilhafterweise ergänzt durch einen Maskierungsaktivator, vorzugsweise EDTA.

2. Siliconelastomer-System nach Anspruch 1, **dadurch gekennzeichnet, daß** das biozide Mittel (II) aus der Gruppe von Vorläufern von aktivem Chlor gewählt wird, die umfaßt:

   - Chloramin B (Natrium-N-chlorbenzol-sulfonamid),
   - Dichloramin T (N,N-Dichlor-p-toluol-sulfonamid),
   - N-Trichlormethylmercapto-4-cyclohexen-1,2-dicarboxylamid,
   - Halazon (Benzoesäure-p-n-dichlorsulfonamid),
   - N-Chlorsuccinid,
   - Trichlormelamin,
   - Chlorazodin

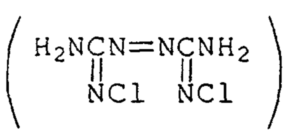

   - die N-Chlor-Derivate von Cyanursäuren, vorzugsweise die Trichlorisocyanursäure und/oder das Natriumdichlorisocyanur-dihydrat,
   - die N-Chlorhydantoine, vorzugsweise das 1-Brom-3-chlor-5,5'-dimethylhydantoin oder das 1,3-Dichlor-5,5'-dimethylhydantoïn,
   - und ihre Mischungen.

3. Siliconelastomer-System nach irgendeinem der vorstehenden Ansprüche, **dadurch gekennzeichnet, daß** die Zusammensetzung POS (I) eine Zweikomponenten-Zusammensetzung A-B ist, Vorläufer von dem Elastomer das

bei Umgebungstemperatur oder bei Erwärmung durch Vermischen der Bestandteile A und B, die isoliert nicht aushärtbar sind, vernetzt.

**4.** Siliconelastomer-System nach irgendeinem der vorstehenden Ansprüche, **dadurch gekennzeichnet, daß** das biozide Mittel (II) frei ist von Inhibitor-Aktivität im Hinblick auf die Vernetzung der Zusammensetzung POS (I).

**5.** Material für die Durchführung von Abformungen, insbesondere von Zähnen, **dadurch gekennzeichnet, daß** es ein System nach irgendeinem der Ansprüche 1 bis 4 umfaßt.

**6.** Material nach Anspruch 5, **dadurch gekennzeichnet, daß** es Zusatzstoffe umfaßt, ausgewählt unter den Süßstoffen, vorzugsweise unter den synthetischen Süßstoffen und/oder den Aromen und/oder den Farbstoffen und/oder den anti-inflammatorischen Stoffen und/oder den isotonischen Produkten, vorzugsweise den Sacchariden und noch mehr bevorzugt den hydrierten Sacchariden und ganz speziell dem Sorbitol und ihren Mischungen.

**7.** Verfahren zur Herstellung des Systems nach irgendeinem der Ansprüche 1 bis 4 und/oder des Materials nach Anspruch 5 oder Anspruch 6, **dadurch gekennzeichnet, daß** es im wesentlichen darin besteht, die Zusammensetzungen des POS (I), das biozide Mittel (II) und das Hilfsmittel (III), vorteilhafterweise ergänzt durch den Maskierungsaktivator, und gegebenenfalls die wie in Anspruch 6 definierten Zusatzstoffe zu vermischen.

**8.** Verwendung des Systems nach einem der Ansprüche 1 bis 4 und/oder des Materials nach Anspruch 5 oder Anspruch 6 für das Abformen, insbesondere von Zähnen, **dadurch gekennzeichnet, daß** sie im wesentlichen darin besteht, in einer solchen Weise vorzugehen, daß die Vernetzunq des Siliconelastomers vorzugsweise durch Vermischen der Bestandteile A und B in Gang gebracht, die Zahnabformung vorgenommen wird und man dann die Vernetzung weiter fortschreiten läßt, bis das Elastomer ausreichend vernetzt wurde und ausreichend hart geworden ist.